(19) Europäisches Patentamt
European Patent Office
Office européen des brevets

(11) EP 0 898 952 B1

(12) **FASCICULE DE BREVET EUROPEEN**

(45) Date de publication et mention de la délivrance du brevet:
**27.11.2002 Bulletin 2002/48**

(51) Int Cl.[7]: **A61K 7/00**, A61K 7/021, A61K 7/42

(21) Numéro de dépôt: **98401985.1**

(22) Date de dépôt: **04.08.1998**

(54) **Utilisation d'un conducteur ionique pour améliorer le photochromisme et composition le contenant**

Verwendung eines Ionenleiters zur Verbesserung der Photochromie und diesen enthaltende Zusammensetzung

Use of an ionic conductor to improve photochromism and composition containing it

(84) Etats contractants désignés:
**DE ES FR GB IT**

(30) Priorité: **26.08.1997 FR 9710658**

(43) Date de publication de la demande:
**03.03.1999 Bulletin 1999/09**

(73) Titulaire: **L'OREAL**
**75008 Paris (FR)**

(72) Inventeur: **Remy, Christophe**
**94400 Vitry/Seine (FR)**

(74) Mandataire: **Dodin, Catherine**
**L'OREAL-DPI**
**6 rue Bertrand Sincholle**
**92585 Clichy Cédex (FR)**

(56) Documents cités:
**FR-A- 2 209 717** **US-A- 4 724 138**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 898 952 B1

## Description

La présente invention se rapporte à l'amélioration des propriétés photochromes d'un composé minéral initialement photochrome, et à son application dans le domaine des compositions cosmétiques notamment.

Les compositions cosmétiques, notamment les compositions de maquillage telles que les poudres libres ou compactes, les fonds de teint, les fards à joues ou à paupières, les rouges à lèvres ou les vernis à ongles, sont constituées d'un véhicule approprié et de différents agents de coloration destinés à conférer une certaine couleur auxdites compositions avant et/ou après leur application sur la peau, les muqueuses, les semi-muqueuses et/ou les phanères telles que les ongles ou les cheveux.

Pour créer des couleurs, on utilise aujourd'hui une gamme d'agents de coloration assez limitée et notamment des laques, des pigments minéraux ou des pigments nacrés. Les laques permettent d'obtenir des couleurs vives, mais pour la plupart sont instables à la lumière, à la température ou au pH. Certaines présentent également l'inconvénient de tacher la peau de manière disgracieuse après application, par dégorgement du colorant. Les pigments minéraux, en particulier les oxydes minéraux sont au contraire très stables mais donnent des couleurs plutôt ternes et pâles.

Pour obtenir des effets colorés, on peut encore employer des pigments nacrés de couleurs variées, mais jamais intenses, qui permettent d'obtenir des effets irisés mais le plus souvent assez faibles.

Il a alors été proposé d'utiliser des composés photochromes dans les compositions de maquillage ou capillaire, de manière à obtenir des changements agréables et variables dans le 'rendu couleur' des maquillages de la peau et/ou des cheveux.

Les composés photochromes sont des composés qui possèdent la propriété de changer de couleur lorsqu'ils sont irradiés par une source lumineuse, puis de reprendre leur couleur initiale, ou une couleur proche, lorsque l'on arrête l'irradiation. De tels composés trouvent notamment une application particulièrement intéressante dans les compositions cosmétiques, en particulier dans les compositions de maquillage telles que les fonds de teint et les fards à joues ou à paupières. En effet, on a constaté que le 'rendu maquillage' d'une peau maquillée est différent selon que l'on se trouve en éclairage naturel ou en éclairage artificiel. Ainsi, un maquillage réalisé en éclairage artificiel paraîtra plus clair en lumière naturelle. A l'inverse, un maquillage réalisé à l'extérieur paraîtra plus sombre dans un endroit éclairé artificiellement.

Notamment, il a été proposé d'utiliser des composés photochromes organiques tels que les composés de la famille des spiropyrannes ou des naphtooxazines.

Ces composés photochromes sont particulièrement intéressants dans la mesure où ils permettent d'obtenir un changement rapide de coloration du support sur lequel ils sont appliqués, lorsque ledit support est exposé aux UV par exemple, avec un retour rapide à la couleur initiale lorsque l'exposition aux UV cesse.

On peut ainsi citer le brevet FR1604929 qui décrit des compositions cosmétiques notamment capillaires sous forme d'aérosol, qui contiennent des composés phototropes tels que des nitrobenzylpyridines, des thiosemicarbazones ou des dérivés de spiropyrannes. Après sprayage de ces compositions sur les cheveux et exposition à la lumière du soleil, on obtient une coloration bleu-violet qui redevient jaune pâle dans l'obscurité.

Il a également été proposé, par exemple par le brevet EP359909, des compositions cosmétiques comprenant des composés photochromes minéraux particulier, choisis parmi les oxydes métalliques, leurs hydrates et leurs complexes. En particulier, ce document mentionne l'utilisation d'oxyde de titane traité de manière à le rendre photochrome, dans des compositions de maquillage telles que des poudres et des fonds de teint.

Toutefois, on a constaté que ces composés photochromes, notamment minéraux, bien qu'ils permettent d'obtenir un maquillage qui semble rester de couleur constante quel que soit l'éclairage, ne permettent toutefois pas d'obtenir un véritable changement de la couleur du maquillage, ou, en d'autres termes, un véritable changement du 'rendu maquillage'.

D'autre part, on a également constaté qu'après l'arrêt de l'irradiation lumineuse, la couleur du maquillage ne revenait pas toujours d'une manière acceptable à sa couleur initiale, en particulier qu'elle ne revenait pas complètement à une couleur identique à ladite couleur initiale.

L'invention a pour but de proposer un procédé particulier permettant l'amélioration du photochromisme, c'est-à-dire des propriétés photochromes, d'un composé minéral initialement photochrome.

Les propriétés photochromes d'un composé peuvent être caractérisées à l'aide des coordonnées trichromatiques (L, a, et b) de la manière décrite avant les exemples. Ces coordonnées permettent notamment la détermination des paramètres $\Delta E30$ et $\Delta(\Delta E)$ qui serviront dans la présente demande à caractériser le photochromisme des composés selon l'invention et hors invention.

D'une manière générale, plus le paramètre $\Delta E30$ est élevé, plus le composé est susceptible de changer de couleur après irradiation. Plus le paramètre $\Delta(\Delta E)$ est élevé, plus le composé est susceptible de revenir à sa couleur initiale.

Ainsi donc, l'invention a pour but notamment d'améliorer les paramètres $\Delta E30$ et $\Delta(\Delta E)$ de la composition, c'est-à-dire d'obtenir les paramètres les plus élevés possibles.

La présente invention a donc pour objet l'utilisation d'un conducteur ionique dans une composition comprenant

un composé photochrome minéral, pour améliorer le photochromisme de ladite composition.
L'invention a également pour objet une composition comprenant au moins un composé photochrome minéral et au moins un conducteur ionique.

Un avantage de l'invention est de permettre d'utiliser, dans les compositions cosmétiques selon l'invention, une quantité de composés photochromes plus faible que celle utilisée dans l'art antérieur, tout en obtenant un effet de maquillage et de couvrance comparable.
Un autre avantage de l'invention est de permettre l'obtention, à quantité de composés photochromes identique, de propriétés photochromes améliorées c'est-à-dire d'un changement de couleur plus important et/ou d'un retour vers la couleur initiale également plus important.

Sans être tenu par la présente explication, le mécanisme permettant l'amélioration des propriétés photochromes d'un composé donné, peut être le suivant. L'on considère un oxyde de titane photochrome dopé au fer. Lors d'une irradiation UV, on peut penser que le cation $Fe^{3+}$ va céder un électron à une entité X qui va se transformer en entité $X^-$, responsable du changement de couleur dudit composé photochrome. On peut supposer qu'ensuite, lors d'une deuxième phase, des électrons de la bande de valence du titane vont se déplacer vers la bande de conduction, générant en conséquence, d'une part des électrons libres susceptibles d'être captés par X pour former $X^-$, d'autre part des lacunes électroniques dans la bande de valence, aussi appelées 'trou' positif, c'est-à-dire un état vacant d'une bande d'énergie, correspondant à une zone avec une charge négative en moins.

L'invention consiste à favoriser les transferts d'électrons au sein de l'oxyde de titane photochrome de manière à améliorer ses propriétés photochromes.

Par 'conducteur ionique' selon l'invention, on entend tout composé susceptible de se séparer en cation et anion lorsqu'il est mis en solution dans l'eau ou dans un milieu aqueux.

Ledit conducteur ionique est de préférence choisi parmi les sels de métaux alcalins ou alcalino-terreux; on peut en particulier citer les chlorures de sodium, de lithium, de potassium, les sulfates de magnésium ou de calcium, seuls ou en mélange.

Ledit conducteur ionique peut être incorporé dans la composition en une quantité déterminable aisément par l'homme du métier sur la base de ses connaissances générales afin d'obtenir l'effet recherché; cette quantité peut notamment être comprise entre 0,5 et 30% en poids par rapport au poids total de la composition, de préférence en une quantité de 1 à 20% en poids.

La composition comprend par ailleurs au moins un composé minéral photochrome, qui peut être choisi parmi tous les composés photochromes de l'art antérieur susceptibles d'être utilisés dans le domaine d'application envisagé. On peut notamment citer les oxydes ou hydrates métalliques ainsi que les aluminosilicates dopés.

Parmi les oxydes métalliques, les hydrates desdits oxydes et leurs complexes, on peut citer ceux décrits dans le brevet EP359909, et notamment les oxydes de titane, de niobium, de silicium, d'aluminium, de zinc, d'hafnium, de thorium, d'étain, de thallium, de zirconium, de béryllium, de cobalt, de calcium, de magnésium. Les oxydes et hydrates d'oxydes de titane, aluminium, zinc, zirconium, calcium et magnésium sont préférés.
De manière plus préférentielle, on utilisera le dioxyde de titane qui peut être rendu photochrome à l'aide d'un métal choisi parmi le fer, le chrome, le cuivre, le nickel, le manganèse, le cobalt, le molybdène, tel quel ou sous forme de sel tel qu'un sulfate, un chlorate, un nitrate, un acétate.

Parmi les aluminosilicates dopés, on peut citer ceux décrits dans la demande 96FR-15451 non encore publiée. Ces aluminosilicates ont de préférence une structure de type :

$$R_8Al_6Si_6O_{24}X_n$$

dans laquelle :

- R représente un élément choisi parmi Na, K, Cs, Rb, Li, Ag ou Ca; de préférence R représentant Na; et
- X représente un ou plusieurs éléments dopants tels que définis ci-dessus,
- n étant compris entre 1 et 5, de préférence compris entre 1 et 3.

On peut en particulier citer parmi ces composés, ceux de la famille des sodalites qui ont pour formule :

$$Na_8Al_6Si_6O_{24}X_2,$$

dans laquelle $X_2$ représente un ou plusieurs anions halogènes, et notamment $Cl_2$, ClBr, $I_2$ ou $Br_2$.

Le composé photochrome peut être incorporé dans la composition en une quantité déterminable aisément par l'homme du métier sur la base de ses connaissances générales, et qui peut notamment être comprise entre 0,01

et 30% en poids par rapport au poids total de la composition, de préférence en une quantité de 1 à 15% en poids.

La composition selon l'invention peut se présenter, de préférence, sous forme d'une composition cosmétique, notamment sous la forme d'un produit à appliquer sur les muqueuses, les semi-muqueuses et/ou les tissus kératiniques tels que la peau et les phanères (ongles, cils, sourcils, poils et cheveux).

En particulier, cette composition peut être un produit de soin et/ou de maquillage de la peau, un produit solaire ou autobronzant, voire un produit capillaire.
Les composés selon l'invention trouvent une application particulière dans le domaine des rouges à lèvres, des fonds de teint, des fards à joues ou à paupières, des eye-liners, des mascaras et des vernis à ongles notamment aqueux, hydroal-cooliques ou solvant.

La composition selon l'invention contient donc un milieu cosmétiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.
Ledit milieu peut comprendre ou se présenter sous la forme de, notamment, une suspension, une dispersion, une solution en milieu aqueux ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray.
L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.

De préférence, la composition selon l'invention comprend une phase aqueuse, et peut donc notamment se présenter sous forme de dispersion, d'émulsion, de lotion, de gel ou de solution aqueuse ou hydroalcoolique.
Toutefois la présence d'une phase aqueuse ou hydrophile n'est pas une nécessité à la réalisation de l'invention; ainsi l'invention peut également être réalisée dans une composition anhydre.

Ainsi, la composition selon l'invention peut comprendre une phase aqueuse, qui peut comprendre de l'eau, une eau florale telle que l'eau de bleuet et/ou une eau minérale telle que l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Lucas, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains.

Ladite phase aqueuse peut comprendre de 0 % à 14 % en poids, par rapport au poids total de la phase aqueuse, d'un monoalcool inférieur en $C_2$-$C_6$ et/ou d'un polyol tel que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition.
Parmi les tensioactifs anioniques qui peuvent être utilisés seuls ou en mélange, on peut citer en particulier les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, $\alpha$-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoyiphosphates/alcoylétherphosphates, acylsarcosinates, alcoylpolypeptidates, alcoylamidopolypeptidâtes, acyliséthionates, alcoyllaurates. Le radical alcoyle ou acyle dans tous ces composés désigne généralement une chaîne de 12 à 18 atomes de carbone. On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques répondant à la formule : $Alk-(OCH_2-CH_2)_n-OCH_2-COOH$ sous forme acide ou salifiée où le substituant Alk correspond à une chaîne linéaire ayant de 12 à 18 atomes de carbone et où n est un nombre entier compris entre 5 et 15.
Parmi les tensioactifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose; les alkylpolyglycosides et les alkylamides des sucres aminés; les produits de condensation d'un $\alpha$-diol, d'un monoalcool, d'un alcoylphénol, d'un amide ou d'un diglycolamide avec le glycidol ou un précurseur de glycidol.

La composition selon l'invention peut également comprendre 0 à 5 % en poids, par rapport au poids total de

l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations préférentielles allant de 0 à 6 % en poids, par rapport au poids total de l'émulsion. L'agent épaississant peut être choisi parmi:

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxy-méthylcellulose, les dérivés de l'amidon, les dérivés d'éthers de cellulose possédant des groupes ammonium quaternaires, les polysaccharides cationiques;
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque.
Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène.
Ledit polymère peut être choisi parmi la nitrocellulose, l'acétobutyrate de cellulose, les butyralpolyvinyliques, les résines alkydes, les polyesters, les acryliques, les vinyliques, et/ou les polyuréthannes.
On peut en particulier citer les copolymères d'acide (méth)acrylique et d'au moins un monomère ester d'acide (méth) acrylique linéaire, ramifié ou cyclique et/ou d'au moins un monomère amide d'acide (méth)acrylique mono ou di-substitué linéaire, ramifié ou cyclique; les copolymères acide (méth)acrylique/(meth)acrylate de tertio-butyle et/ou (méth) acrylate d'isobutyle/(méth)acrylate d'alkyle en $C_1$-$C_4$; les terpolymères et tétrapolymères acide (meth)acrylique/acrylate d'éthyle/ méthacrylate de méthyle; les tétrapolymères méthacrylate de méthyle/acrylate de butyle ou d'éthyle/acrylate ou méthacrylate d'hydroxyéthyle ou de 2-hydroxypropyle/acide (meth)acrylique; les copolymères d'acide acrylique et de méthacrylate d'alkyle en $C_1$-$C_4$ ; les terpolymères de vinyl pyrrolidone, d'acide acrylique et de méthacrylate d'alkyle en $C_{1-20\,;}$ les copolymères amphotères; les esters vinyliques d'acide ramifiés; les esters vinyliques d'acide benzoïque; les copolymères d'acide (meth)acrylique et d'au moins un monomère oléfinique; les copolymères de monoacide vinylique et/ou de monoacide allylique.
Parmi les résines, on peut citer les résines du type aryl-sulfonamide formaldéhyde ou aryl-sulfonamide époxy; les résines du type acrylique, styrénique, acrylate-styrénique et vinylique.

La composition peut également comprendre au moins un plastifiant, tel que le phosphate de tricrésyle, le benzoate de benzyle, le citrate de triéthyle, le citrate de tributyle, l'acétylcitrate de triéthyle, l'acétylcitrate de triétyl-2 hexyle, le camphre; les éthers de glycol; l'huile de ricin oxyéthylénée à 40 moles d'oxyde d'éthylène; le propylène glycol; le butyl glycol ; l'éthylène glycol monométhyléther acétate; les éthers de propylène glycol; les éthers esters de propylène glycol et d'éthylène glycol; les esters de diacides tels que les phtalates et adipates de diéthyle, dibutyle et de diisopropyle, les tartrates de diéthyle et dibutyle, les succinates de diéthyle et de dibutyle, les sébacates de diéthyle et de dibutyle, les phosphates de diéthyle, de dibutyle et de diéthyl-2 hexyle, l'acétyl citrate de diéthyle ou de dibutyle; les esters de glycérol. Les plastifiants peuvent être généralement présents à une teneur allant de 1 % à 40 % en poids par rapport au poids total de la composition.

La composition selon l'invention. peut également comprendre une phase grasse, notamment constituée de corps gras liquides à 25°C, tels que des huiles d'origine animale, végétale, minérale ou synthétique; de corps gras solides à 25°C tels que des cires d'origine animale, végétale, minérale ou synthétique; de corps gras pâteux; de gommes; de leurs mélanges.

Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa). De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair

est de l'ordre de 40-100°C.
On peut ainsi citer les huiles siliconées volatiles, telles que

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexylheptaméthyltrisiloxane ou de l'octylheptaméthyltrisiloxane.

On peut également citer les huiles volatiles hydrocarbonées, telles que les isoparaffines et notamment l'isododécane; et des huiles fluorées comme celle commercialisée sous la dénomination GALDEN® (MONTEFLUOS).
On peut également utiliser des huiles non volatiles, parmi lesquelles on peut citer:

- les polyalkyl($C_1$-$C_{20}$) siloxanes et notamment ceux à groupements terminaux triméthylsilyle, de préférence ceux dont la viscosité est inférieure à 0,06 $m^2/s$ parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule

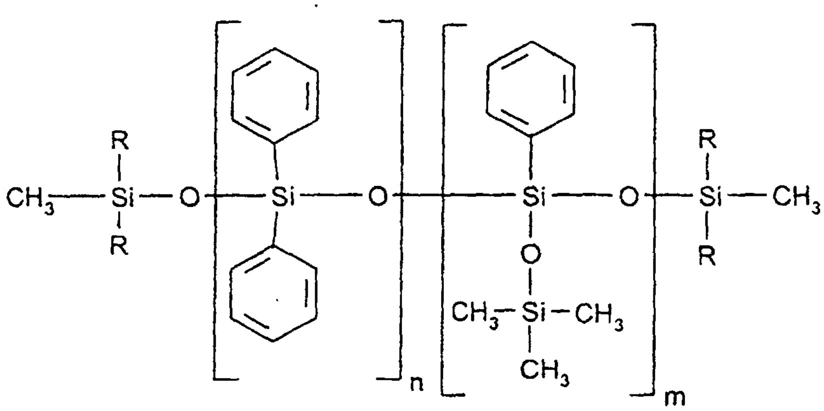

  dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges.
On peut notamment citer :

- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cirés fluorées.

La composition selon l'invention peut également comprendre un ou plusieurs solvants organiques cosmétiquement acceptables (tolérance, toxicologie et toucher acceptables). Ces solvants organiques peuvent représenter de 0 % à 98 % du poids total de la composition et peuvent être choisis dans le groupe constitué par les solvants organiques hydrophiles, les solvants organiques lipophiles, les solvants amphiphiles ou leurs mélanges.
Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone ; les éthers de glycol comme le diéthylène glycol mono-méthyl ou mono-éthyl éther et les éthers de propylène glycol comme le dipropylène glycol méthyl éther. Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate. Comme solvants organiques lipophiles, on peut citer par exemple les esters gras tels que l'adipate de diisopropyle, l'adipate de dioctyle, les benzoates d'alkyle, le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl-décyle, le palmitate de 2-octyl-décyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine.
La composition peut comprendre en outre une phase particulaire, qui peut comprendre des pigments et/ou des nacres et/ou des charges habituellement utilisés dans les compositions cosmétiques.
Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.
Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique. On peut citer les dioxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium, le noir de carbone. On peut encore citer les laques couramment employées pour conférer aux lèvres et à la peau un effet de maquillage, qui sont des sels de calcium, de baryum, d'aluminium ou de zirconium.
Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Coming) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.
La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.
La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des acides gras essentiels, des sphingolipides, des agents autobronzants tels que la DHA, des filtres solaires, des agents antimousses, des agents séquestrants, des antioxydants.
Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

Les compositions cosmétiques selon l'invention sont essentiellement celles concernant le maquillage du visage, c'est-à-dire les fards à paupières ou à joues, les eye-liners, les mascaras, les poudres, les fonds de teint, les crèmes teintées, les rouges à lèvres, mais également le maquillage des cheveux notamment des gels, crèmes ou mousses pour la coloration temporaire des cheveux, et le maquillage des ongles notamment les vernis à ongles aqueux.
L'invention est illustrée plus en détail dans les exemples suivants.

Méthode de détermination du paramètre ΔE à l'aide des coordonnées trichromatiques.

Les propriétés photochromes d'un composé peuvent être caractérisées de la manière suivante, à l'aide des coordonnées trichromatiques (L, a, et b).
Le composé en poudre est tassé dans une coupelle métallique.
On mesure les coordonnées trichromatiques (L0, a0, b0) de la poudre tassée à l'aide d'un colorimètre Minolta CR300. On irradie la coupelle pendant 30 minutes à l'aide d'une lampe émettant dans la totalité du spectre solaire (lampe au Xénon, Suntest CPS de marque Héraeus, reproduisant le spectre solaire), puis l'on mesure les nouvelles coordonnées (L30, a30 et b30) qui reflètent le changement de couleur suite à l'irradiation.
On détermine le paramètre ΔE30 de la manière suivante :

$$\Delta E30 = [\,(L30 - L0)^2 + (a30 - a0)^2 + (b30 - b0)^2]^{1/2}$$

Puis, on place le composé pendant 30 minutes dans l'obscurité complète et l'on mesure à nouveau le changement de couleur. On détermine le paramètre ΔE60 de la manière suivante :

$$\Delta E60 = [\,(L60 - L0)^2 + (a60 - a0)^2 + (b60 - b0)^2]^{1/2}$$

La valeur Δ(ΔE), égale à la valeur absolue de la différence entre ΔE60 et ΔE30, reflète la faculté d'un composé à revenir, après irradiation et obscurité, à une couleur proche de celle de l'état initial, c'est-à-dire avant irradiation.

Exemple 1

Le composé photochrome pris comme exemple est l'oxyde de titane dopé au fer vendu par C.C.I.C. par l'intermédiaire d'IKEDA, sous la dénomination 'PHOTOGENICA 1'.
Ses caractéristiques initiales sont les suivantes : ΔE30 = 10,3 et Δ(ΔE) = 2,4
On prépare un mélange comprenant 10% en poids de chlorure de sodium et 90% en poids d'oxyde de titane dopé au fer. On mesure les caractéristiques du mélange selon la méthode ci-dessus.
On obtient les résultats donnés dans le tableau ci-après.

| | ΔE30 | Δ(ΔE) |
|---|---|---|
| oxyde de titane dopé au fer | 10,3 | 2,4 |
| 90% oxyde de titane dopé au fer +10% NaCl | 12,0 | 4,6 |

On constate donc qu'un ajout de 10% de chlorure de sodium permet d'obtenir un gain de plus de 15% sur le changement de couleur de l'oxyde de titane; on obtient également plus de 90% de réversibilité du phénomène de photochromisme.

Exemple 2

Selon l'exemple 1, on mesure les caractéristiques des mélanges ci-dessous.

| | ΔE30 |
|---|---|
| oxyde de titane dopé au fer + 10 % glycérine | 17,1 |
| oxyde de titane dopé au fer + 10% glycérine + 10% LiCl | 20,9 |

L'ajout de chlorure de lithium permet d'obtenir un gain de plus de 20% sur le changement de couleur.

Exemple 3

On prépare une poudre ayant la composition suivante :

- talc 30 g
- mica 20 g
- poudre de Nylon 16 g
- stéarate de zinc 4 g
- oxyde de fer 2 g
- oxyde de titane dopé au fer photochrome 10 g
- oxychlorure de bismuth 10 g
- liant gras 8 g

On obtient une composition présentant des propriétés photochromes caractérisées par un ΔE30 de 2,5.
Lorsqu'on ajoute 15 g de chlorure de sodium à cette composition, on obtient une composition ayant de bonnes propriétés cosmétiques et présentant un ΔE30 supérieur de plus de 50% à celui de la composition précédente.

**Revendications**

1. Utilisation d'un conducteur ionique dans une composition comprenant un composé photochrome minéral, pour améliorer le photochromisme de ladite composition.

2. Utilisation selon la revendication 1, dans laquelle ledit conducteur ionique est choisi parmi les sels de métaux alcalins ou alcalino-terreux.

3. Utilisation selon l'une des revendications précédentes, dans laquelle le conducteur ionique est choisi parmi les chlorures de sodium, de lithium, de potassium, les sulfates de magnésium ou de calcium, seuls ou en mélange.

4. Utilisation selon l'une des revendications précédentes, dans laquelle le conducteur ionique est incorporé dans la composition en une quantité comprise entre 0,5 et 30% en poids par rapport au poids total de la composition, de préférence en une quantité de 1 à 20% en poids.

5. Utilisation selon l'une des revendications précédentes, dans laquelle le composé minéral photochrome est choisi parmi les oxydes ou hydrates métalliques et les aluminosilicates dopés.

6. Utilisation selon l'une des revendications précédentes, dans laquelle le composé minéral photochrome est choisi parmi les oxydes de titane, de niobium, de silicium, d'aluminium, de zinc, d'hafnium, de thorium, d'étain, de thallium, de zirconium, de béryllium, de cobalt, de calcium, de magnésium.

7. Utilisation selon l'une des revendications précédentes, dans laquelle le composé minéral photochrome est incorporé dans la composition en une quantité comprise entre 0,01 et 30% en poids par rapport au poids total de la composition, de préférence en une quantité de 1 à 15% en poids.

8. Utilisation selon l'une des revendications précédentes, dans laquelle la composition comprend une phase aqueuse.

9. Utilisation selon l'une des revendications précédentes, dans laquelle la composition se présente sous forme de dispersion, d'émulsion, de lotion, de gel ou de solution aqueuse ou hydroalcoolique.

10. Utilisation selon l'une des revendications précédentes, dans laquelle la composition se présente sous forme d'une composition cosmétique, notamment sous la forme d'un produit à appliquer sur les muqueuses, les semi-muqueuses et/ou les tissus kératiniques tels que la peau et les phanères.

11. Utilisation selon l'une des revendications précédentes, dans laquelle la composition se présente sous forme d'un produit de soin et/ou de maquillage de la peau tel qu'un rouge à lèvres, un fond de teint, un fard à joues ou à paupières, un eye-liner, un mascara, une poudre, un vernis à ongles; d'un produit solaire ou autobronzant, d'un produit capillaire.

12. Composition comprenant au moins un composé photochrome minéral et au moins un conducteur ionique.

13. Composition selon la revendication 12, dans laquelle le conducteur ionique est choisi parmi les sels de métaux alcalins ou alcalino-terreux.

14. Composition selon l'une des revendications 12 à 13, dans laquelle le conducteur ionique est choisi parmi les chlorures de sodium, de lithium, de potassium, les sulfates de magnésium ou de calcium, seuls ou en mélange.

15. Composition selon l'une des revendications 12 à 14, dans laquelle le conducteur ionique est présent en une quantité comprise entre 0,5 et 30% en poids par rapport au poids total de la composition, de préférence en une quantité de 1 à 20% en poids.

16. Composition selon l'une des revendications 12 à 15, dans laquelle le composé minéral photochrome est choisi parmi les oxydes ou hydrates métalliques, et les aluminosilicates dopés.

17. Composition selon l'une des revendications 12 à 16, dans laquelle le composé minéral photochrome est choisi parmi les oxydes de titane, de niobium, de silicium, d'aluminium, de zinc, d'hafnium, de thorium, d'étain, de thallium, de zirconium, de béryllium, de cobalt, de calcium, de magnésium.

18. Composition selon l'une des revendications 12 à 17, dans laquelle le composé minéral photochrome est présent en une quantité comprise entre 0,01 et 30% en poids par rapport au poids total de la composition, de préférence en une quantité de 1 à 15% en poids.

19. Composition selon l'une des revendications 12 à 18, comprenant une phase aqueuse.

20. Composition selon l'une des revendications 12 à 19, se présentant sous la forme d'une suspension, une dispersion, une solution en milieu aqueux ou hydroalcoolique, une émulsion huile-dans-eau, eau-dans-huile, ou multiple; un gel ou une mousse; un gel émulsionné; une dispersion de vésicules notamment lipidiques; une lotion biphase ou multiphase; un spray.

21. Composition selon l'une des revendications 12 à 20, se présentant sous forme d'une composition cosmétique, notamment sous la forme d'un produit à appliquer sur les muqueuses, les semi-muqueuses et/ou les tissus kératiniques tels que la peau et les phanères.

22. Composition selon l'une des revendications 12 à 21, se présentant sous forme d'un produit de soin et/ou de maquillage de la peau tel qu'un rouge à lèvres, un fond de teint, un fard à joues ou à paupières, un eye-liner, un mascara, une poudre, un vernis à ongles; d'un produit solaire ou autobronzant, d'un produit capillaire.

**Claims**

1. Use of an ionic conductor in a composition comprising an inorganic photochromic compound, in order to improve the photochromism of the said composition.

2. Use according to Claim 1, in which the said ionic conductor is selected from the salts of alkali or alkaline-earth metals.

**3.** Use according to one of the preceding claims, in which the ionic conductor is selected from the chlorides of sodium, lithium, potassium, and the sulphates of magnesium or calcium, alone or as a mixture.

**4.** Use according to one of the preceding claims, in which the ionic conductor is incorporated into the composition in an amount of between 0.5 and 30% by weight relative to the total weight of the composition, preferably in an amount of 1 to 20% by weight.

**5.** Use according to one of the preceding claims, in which the photochromic inorganic compound is selected from metal oxides or hydrates and doped aluminosilicates.

**6.** Use according to one of the preceding claims, in which the photochromic inorganic compound is selected from the oxides of titanium, niobium, silicon, aluminium, zinc, hafnium, thorium, tin, thallium, zirconium, beryllium, cobalt, calcium and magnesium.

**7.** Use according to one of the preceding claims, in which the photochromic inorganic compound is incorporated into the composition in an amount of between 0.01 and 30% by weight relative to the total weight of the composition, preferably in an amount of 1 to 15% by weight.

**8.** Use according to one of the preceding claims, in which the composition comprises an aqueous phase.

**9.** Use according to one of the preceding claims, in which the composition is in the form of a dispersion, emulsion, lotion, gel or aqueous or aqueous-alcoholic solution.

**10.** Use according to one of the preceding claims, in which the composition is in the form of a cosmetic composition, in particular in the form of a product to be applied to the mucous membranes, the mucocutaneous tissues and/or the keratinous tissues, such as the skin and parts of the exoskeleton.

**11.** Use according to one of the preceding claims, in which the composition is in the form of a care and/or make-up product for the skin, such as a lipstick, a foundation, a blusher or eye-shadow, an eye-liner, a mascara, a powder, a nail varnish; a suncare or self-tanning product, or a haircare product.

**12.** Composition comprising at least one inorganic photochromic compound and at least one ionic conductor.

**13.** Composition according to Claim 12, in which the ionic conductor is selected from the salts of alkali or alkaline-earth metals.

**14.** Composition according to one of Claims 12 and 13, in which the ionic conductor is selected from the chlorides of sodium, lithium, potassium, and the sulphates of magnesium or calcium, alone or as a mixture.

**15.** Composition according to one of Claims 12 to 14, in which the ionic conductor is present in an amount of between 0.5 and 30% by weight relative to the total weight of the composition, preferably in an amount of 1 to 20% by weight.

**16.** Composition according to one of Claims 12 to 15, in which the photochromic inorganic compound is selected from metal oxides or hydrates and doped aluminosilicates.

**17.** Composition according to one of Claims 12 to 16, in which the photochromic inorganic compound is selected from the oxides of titanium, niobium, silicon, aluminium, zinc, hafnium, thorium, tin, thallium, zirconium, beryllium, cobalt, calcium and magnesium.

**18.** Composition according to one of Claims 12 to 17, in which the photochromic inorganic compound is present in an amount of between 0.01 and 30% by weight relative to the total weight of the composition, preferably in an amount of 1 to 15% by weight.

**19.** Composition according to one of Claims 12 to 18, comprising an aqueous phase.

**20.** Composition according to one of Claims 12 to 19, which is in the form of a suspension, a dispersion or a solution in an aqueous or aqueous-alcoholic medium; an oil-in-water, water-in-oil or multiple emulsion; a gel or a foam; an emulsified gel; a dispersion of vesicles, in particular lipid vesicles; a two-phase or multi-phase lotion; or a spray.

21. Composition according to one of Claims 12 to 20, which is in the form of a cosmetic composition, in particular in the form of a product to be applied to the mucous membranes, the mucocutaneous tissues and/or the keratinous tissues, such as the skin and parts of the exoskeleton.

22. Composition according to one of Claims 12 to 21, which is in the form of a care and/or make-up product for the skin, such as a lipstick, a foundation, a blusher or eye-shadow, an eye-liner, a mascara, a powder, a nail varnish; a suncare or self-tanning product, or a haircare product.

**Patentansprüche**

1. Verwendung eines Ionenleiters in einer Zusammensetzung, die eine anorganische, photochrome Verbindung enthält, zur Verbesserung der Photochromie dieser Zusammensetzung.

2. Verwendung nach Anspruch 1, wobei der Ionenleiter unter den Salzen von Alkalimetallen und den Salzen von Erdalkalimetallen ausgewählt ist.

3. Verwendung nach einem der vorhergehenden Ansprüche, wobei der Ionenleiter unter Natriumchlorid, Lithiumchlorid, Kaliumchlorid, Magnesiumsulfat, Calciumsulfat und deren Gemischen ausgewählt ist.

4. Verwendung nach einem der vorhergehenden Ansprüche, wobei der Innenleiter in einem Mengenanteil von 0,5 bis 30 Gew.-%, bezogen auf das Gesamtgewicht der Zusammensetzung, und vorzugsweise 1 bis 20 Gew.-% in die Zusammensetzung eingearbeitet wird.

5. Verwendung nach einem der vorhergehenden Ansprüche, wobei die anorganische, photochrome Verbindung unter den Metalloxiden, Metallhydraten und dotierten Aluminiumsilicaten ausgewählt ist.

6. Verwendung nach einem der vorhergehenden Ansprüche, wobei die anorganische, photochrome Verbindung unter den oxiden von Titan, Niob, Silicium, Aluminium, Zink, Hafnium, Thorium, Zinn, Thallium, Zirconium, Beryllium, Cobalt, Calcium und Magnesium ausgewählt ist.

7. Verwendung nach einem der vorhergehenden Ansprüche, wobei die anorganische, photochrome Verbindung in die Zusammensetzung in einem Mengenanteil von 0,01 bis 30 Gew.-%, bezogen auf das Gesamtgewicht der Zusammensetzung, und vorzugsweise 1 bis 15 Gew.-% eingearbeitet wird.

8. Verwendung nach einem der vorhergehenden Ansprüche, wobei die Zusammensetzung eine wäßrige Phase enthält.

9. Verwendung nach einem der vorhergehenden Ansprüche, wobei die Zusammensetzung als Dispersion, Emulsion, Lotion, Gel oder wäßrige oder wäßrig-alkoholische Lösung vorliegt.

10. Verwendung nach einem der vorhergehenden Ansprüche, wobei die Zusammensetzung in Form einer kosmetischen Zusammensetzung und insbesondere in Form eines Produktes vorliegt, das auf die Schleimhäute, Semischleimhäute und/oder Keratinsubstanzen, wie die Haut und die Hautanhangsgebilde, aufgebracht werden soll.

11. Verwendung nach einem der vorhergehenden Ansprüche, wobei die Zusammensetzung in Form eines Produktes zur Pflege und/oder zum Schminken der Haut, beispielsweise als Lippenstift, Make-up, Wangenrouge, Lidschatten, Eyeliner, Mascara, Puder und Nagellack, in Form eines Sonnenschutzmittels, Selbstbräunungsmittels oder Produktes zur Haarbehandlung vorliegt.

12. Zusammensetzung, die mindestens eine anorganische, photochrome Verbindung und mindestens einen Innenleiter enthält.

13. Zusammensetzung nach Anspruch 12, wobei der Ionenleiter unter den Salzen von Alkalimetallen und den Salzen von Erdalkalimetallen ausgewählt ist.

14. Zusammensetzung nach einem der Ansprüche 12 bis 13, wobei der Innenleiter unter Natriumchlorid, Lithiumchlorid, Kaliumchlorid, Magnesiumsulfat, Calciumsulfat und deren Gemischen ausgewählt ist.

**15.** Zusammensetzung nach einem der Ansprüche 12 bis 14, wobei der Innenleiter in einem Mengenanteil von 0,5 bis 30 Gew.-%, bezogen auf das Gesamtgewicht der Zusammensetzung, und vorzugsweise 1 bis 20 Gew.-% vorliegt.

**16.** Zusammensetzung nach einem der Ansprüche 12 bis 15, wobei die anorganische, photochrome Verbindung unter den Metalloxiden, Metallhydraten und dotierten Aluminiumsilicaten ausgewählt ist.

**17.** Zusammensetzung nach einem der Ansprüche 12 bis 16, wobei die anorganische, photochrome Verbindung unter den Oxiden von Titan, Niob, Silicium, Aluminium, Zink, Hafnium, Thorium, Zinn, Thallium, Zirconium, Beryllium, Cobalt, Calcium und Magnesium ausgewählt ist.

**18.** Zusammensetzung nach einem der Ansprüche 12 bis 17, wobei die anorganische photochrome Verbindung in einem Mengenanteil von 0,01 bis 30 Gew.-%, bezogen auf das Gesamtgewicht der Zusammensetzung, und vorzugsweise 1 bis 15 Gew.-% vorliegt.

**19.** Zusammensetzung nach einem der Ansprüche 12 bis 18, die eine wäßrige Phase enthält.

**20.** Zusammensetzung nach einem der Ansprüche 12 bis 19, die in Form einer Suspension, einer Dispersion, einer Lösung in einem wäßrigen oder einem wäßrig-alkoholischen Medium, einer Öl-in-Wasser-Emulsion, einer Wasser-in-Öl-Emulsion oder einer multiplen Emulsion; eines Gels oder eines Schaums; eines emulgierten Gels, einer Vesikeldispersion, insbesondere einer Lipidvesikeldispersion; einer Zweiphasenlotion oder einer Mehrphasenlotion, oder eines Sprays vorliegt,

**21.** Zusammensetzung nach einem der Ansprüche 12 bis 20, die in Form einer kosmetischen Zusammensetzung und insbesondere in Form eines Produktes vorliegt, das auf die Schleimhäute, Semischleimhäute und/oder Keratinsubstanzen, wie die Haut und die Hautanhangsgebilde, aufgebracht werden soll.

**22.** Zusammensetzung nach einem der Ansprüche 12 bis 21, wobei die Zusammensetzung in Form eines Produktes zur Pflege und/oder zum Schminken der Haut, beispielsweise als Lippenstift, Make-up, Wangenrouge, Lidschatten, Eyeliner, Mascara, Puder und Nagellack, in Form eines Sonnenschutzmittels, Selbstbräunungsmittels oder Produktes zur Haarbehandlung vorliegt.